Europäisches Patentamt

(19) European Patent Office

Office européen des brevets

(11) Numéro de publication: **0 083 267**
**B1**

(12) **FASCICULE DE BREVET EUROPEEN**

(45) Date de publication du fascicule du brevet:
08.05.85

(51) Int. Cl.⁴: **C 12 N 11/04,** C 12 N 1/04,
C 05 F 11/08

(21) Numéro de dépôt: 82402300.6

(22) Date de dépôt: 15.12.82

(54) Procédé de préparation d'inoculums à faible activité de l'eau à resistance améliorée à la température et à la réhydratation.

(30) Priorité: 29.12.81 FR 8124403

(43) Date de publication de la demande:
06.07.83 Bulletin 83/27

(45) Mention de la délivrance du brevet:
08.05.85 Bulletin 85/19

(84) Etats contractants désignés:
DE FR GB IT SE

(56) Documents cités:
EP - A - 0 017 565
FR - A - 2 303 080
FR - A - 2 469 861
US - A - 3 168 796
US - A - 3 616 236

BIOLOGICAL ABSTRACTS, vol. 69, no. 8, 1980, page
5824, no. 54441, Philadelphia, USA J.J.
PENA-CABRIALES et al.: "Survival of Rhizobium in soils
undergoing drying"
CHEMICAL ABSTRACTS, vol. 80, no. 5, 4 févrler 1974,
page 317, no. 25905p, Columbus, Ohio, USA
MICROBIOLOGY ABSTRACTS, serie A: Industrial
microbiology, vol. 4, no. 8, mai 1969, no. A4153, Londres,
G.B.

(73) Titulaire: RHONE-POULENC S.A., 25, qual Paul Doumer,
F-92408 Courbevoie (FR)

(72) Inventeur: Jung, Gérard, rue des Grands Jardins,
Leuville/sur/orge F-91310 Montlhery (FR)
Inventeur: Mugnier, Jacques, 63, rue de Cherche Midi,
F-75006 Paris (FR)

(74) Mandataire: Martin, Henri et al, RHONE-POULENC
RECHERCHES Service Brevets Chimie et
Polymères 25, quai Paul Doumer, F-92408 Courbevoie
Cedex (FR)

**0 083 267**

## Description

La présente invention a pour objet un nouveau procédé de préparation d'inoculums à faible activite de l'eau ainsi que les produits obtenus, lesquels présentent une longue viabilité et une resistance améliorée à la température et à la réhydratation.

Elle a trait plus particulièrement à l'inclusion de micro-organismes du genre Rhizobium dans une matrice constituée par un gel de polymère, à des fins agronomiques.

Depuis de nombreuses années, on a cherché à conserver les Rhizobium par deshydratation à l'aide d'adjuvants divers: sur des billes de porcelaine deshydratées (HUNT, 1958 J. Bacteriol. 76, 453 – 454), dans des huiles liquides deshydratées (Brevet US 3 0345 968), dans de l'huile mélangée avec du talc ou du kaolin (US 31 68 796) sur du plâtre (GB 1 490 046), sur du gypse (FRASER, 1975 J. Appl. Bacteriol. 39, 345), à l'aide de sulfate de sodium (NILSSON 1957 Rev. Sci. Instrum. 11, 212).

Le procédé de deshydratation selon les références ci-dessus est lent et hasardeux ce qui nuit à la survie du micro-organisme (Gault dans Newsletters, vol. 21 (1) Avril 1981, p. 34 et Vincent dans Rhizobium Newsletter, 25, 1981 et d'autres auteurs).

Les étapes conduisant à l'état deshydraté seraient la cause de la destruction du Rhizobium.

On a aussi proposé des procédés tels que la lyophilisation (US n° 3 168 796) ou l'atomisation (OCHIN 1980, thèse Lille).

Mais dans ces deux derniers cas, différentes substances protectrices ou supports sont utilisés lactose, lait, chlorhydrate de cysteine, maltodextrine ou des substances chimiquement inertes et finement divisées du type kaolin, charbon activé.

Mais même avec l'addition de ces substances protectrices, le procédé de séchage, la conservation et la rehydratation sont très difficiles.

La rehydratation est considérée comme un procédé hasardeux.

Il est connu, en effet, que la reprise d'eau progressive par un micro-organisme totalement deshydraté est léthale (Amarger, Arch. Mikrobiol 81, 361 – 366 (1972). Lorsque par exemple une ampoule lyophilisée contenant des micro-organismes est ouverte, en moins de huit jours à l'air ambinat tous les micro-organismes sont morts.

Il est donc possible de conserver une partie des micro-organismes par une deshydration poussée mais lorsqu'il faudra l'utiliser en plein champ, l'emploi d'une solution nécessaire pour la rehydratation va poser de nombreux problèmes.

Le but poursuivi depuis de nombreuses années est d'obtenir un micro-organisme inclus dans un polymère qui puisse se conserver indéfiniment sans conditions spéciales et qui se présente sous une forme solide au moment de son utilisation.

Ce problème étant difficile à résoudre, un préjugé consiste à considérer que pour assurer la survie des micro-organismes, il faut conserver dans les inoculums une certaine humidité.

De cette manière, le milieu de culture est apporté sur un support tel que la tourbe, ou dans un gel polymère tel quel où l'on essaie de réduire la perte en eau, ou tout au moins de maintenir une disponibilité de l'eau (ou une activité de l'eau) dans l'inoculum qui renferme, outre le micro-organisme, des éléments minéraux solubles, une source hydrocarbonée telle que mannitol et une source azotee telle que l'extait de levure.

C'est ainsi que dans le brevet US n° 4 155 737, on a proposé l'inclusion d'un micro-organisme dans un gel de polymère. Selon ce brevet, le gel polymère peut être constitué par un gel de polyacrylamide ou un gel de silice.

Or, l'on sait que pratiquement le polymère doit être biodégradable ou tout au moins non polluant.

C'est pourquoi, dans la demande européenne 17 565 au nom de la demanderesse, on a revendiqué de choisir comme support une matrice à base d'au moins un polymère du groupe des polysaccharides, ce procédé se caractérisant par le fait que l'on fait subir audit gel contenant le micro-organisme un séchage qui laisse subsister au moins une partie de l'eau solvante. Ce séchage peut être amélioré par addition d'une substance à forte absorption d'eau telle qu'une silice synthétique ou naturelle.

En général, afin de s'assurer d'une bonne survie des micro-organismes, l'on maintient une activite de l'eau à une valeur avantageusement supérieure à 0,85. Mais l'on sait que pour les humidités optimales requises dans les inoculums référencés dans ces brevets, par exemple pour la survie des Rhizobium, la germination des spores de champignons et le développement des moisissures et contaminants est à craindre pour des produits non stériles.

Plus généralement pour la plupart des moisissures, l'activité de l'eau, limite au-delà de laquelle un développement microbien est possible est comprise entre 0,80 et 0,95 mais on trouve aussi certaines moisissures capables de se développer à des activités de l'eau plus basses, celles-ci sont dites »xérophiles« ou »osmotolérantes« pour désigner l'ensemble des micro-oganismes capables de se développer dans un milieu à faible activité de l'eau ($a_w = 0,6 – 0,7$).

Il est donc nécessaire pour assurer la stabilité microbiologique des inoculums, pour une teneur en eau optimale, de préparer et de stocker les inoculums stérilement ou d'assurer leur conservation au froid; la stabilité des inoculums réfrigérés résulte à la fois de l'abaissement de la température et du blocage d'une fraction d'eau disponible pour les contaminants; l'intégrité de l'inoculum est respectée, mais au prix des lourdes contraintes de la chaine de froid.

2

En règle générale, ces inoculums demeurent fragiles vis-à-vis des contaminants et résistent mal au-delà d'une quarantaine de degrés, ce qui est un inconvénient dans les pays chauds, ou de manière générale, lors de la conservation du fait d'un risque certain de contamination.

Pour maîtriser les différents processus de dégradation: développement de micro-organismes, réactions chimiques, réactions enzymatiques, altération de la texture, on peut agir plus ou moins sélectivement sur chacun de ces phénomènes en jouant sur les paramètres habituels de la physico-chimie, température, pH, potentiel redox, utilisation d'inhibiteurs spécifiques.

Mais ces moyens se sont avérés limités. Ceci vient en particulier du fait que comme souligné précédemment, l'enseignement de l'art antérieur repose sur le principe que l'eau disponible doit être suffisante pour assurer la survie du micro-organisme.

Des travaux importants de la demanderesse ont mis en évidence que partant des valeurs habituelles de l'activité de l'eau dans les inoculums, de l'ordre de 0,85 et plus, si l'on abaissait l'activité de l'eau, il y aurait bien, dans un premier temps, diminution du développement des moisissures et des réactions enzymatiques, mais que l'on observait une plasmolyse importante des cellules, et des réactions de brunissement.

Le phénomène est similaire lorsque l'on passe d'une activité de l'eau très basse vers une activité de l'eau de l'ordre de 0,85 au plus.

En effet, le passage aux activités de l'eau intermédiaires de l'ordre de 0,4 à 0,8, est léthal pour les bactéries que ce passage soit réalisé à la deshydratation ou à la rehydratation.

Pour la deshydratation on est amené à vaincre ce phénomène par un passage extrêmement rapide de cette zone léthale à l'aide de la lyophilisation (brevet US n° 3 168 796) ou à l'aide de l'atomisation.

Mais cette technique ne résout pas le problème de la rehydratation, dans les conditions d'application où la rehydratation est lente et progressive.

Il est également connu d'après la demande de brevet japonaise 8830/1973 un procédé de production de levures ou de bactéries présentant une bonne stabilité au stockage, caractérisé en ce que on sèche par atomisation des solutions neutres ou légèrement acide contenant: une dispersion bactérienne, une protéine choisie parmi les protéines de soja, la zeine, la mucine, les lactoprotéines, un polymère qui peut être un alginate, un dissacharide ou un sucre à plus haut poids moléculaire et, si nécessaire, un alcool polyhydrique. Cette technique nécessite la mise en oeuvre de grandes quantités d'additifs du type protéine ou polysaccharides.

Le concept de l'invention consiste à extraire du produit la fraction d'eau la plus disponible, à éliminer toute l'eau solvante, (telle que définie par exemple par LEUNG. H. K 1981 Structure and properties of water — Cereal food World 26 7-350-352) et à utiliser les caractéristiques du mélange gel et substances solubles pour éviter de manière tout à fait inattendue pour l'homme du métier l'apparition de ces phénomènes néfastes lors de la rehydratation.

La valeur de l'activité de l'eau au delà de laquelle on n'a plus de développement de moisissures, de réactions enzymatiques et de réactions de brunissement dépend également des conditions du milieu, de la nature du micro-organisme et du gel.

On peut admettre que d'une manière générale, pour les valeurs minimales de l'$a_w$, on conserve un nombre maximum de cellules viables.

Les différentes valeurs de l'activité de l'eau sont obtenues et déterminées par la méthode dite des solutions aqueuses saturées (Multon 1981 Techniques d'analyses et de contrôle dans les industries agro-alimentaires — APRIA —).

Le procédé selon l'invention se caractérisé par le fait qu'on inclut le micro-organisme dans son milieu de culture dans un gel de polysaccharide et que l'on abaisse l'activité de l'eau dans l'inoculum en dessous de 0,1 et qu'on la maintient à cette valeur.

Les différentes valeurs optimales de l'activité de l'eau pour la protection du micro-organisme lors de son stockage et lors de sa rehyratation dépendent notamment de la température, de la nature du micro-organisme, de la nature et de la concentration des composés présents, et du type de polymère utilisé.

La connaissance de la vitesse de destruction des micro-organismes à la température est un paramètre nécessaire pour la mise au point de procédés de préparation très sûrs pour un inoculum. Les travaux de la demanderesse ont montré que la résistance des micro-organismes dans les inoculums aux températures élevées (60° C) était maximale pour les valeurs de l'activité de l'eau les plus faibles et que la perte de la survie intervenait rapidement à ces mêmes températures quand ces valeurs allaient vers 0,5.

Les travaux de la demanderesse ont également montré que lors de la rehydratation, la vitesse de destruction des micro-organismes aux activités de l'eau intermédiaires de l'ordre de 0,4—0,8 est fonction de la source hydrocarbonée du milieu de culture et du gel.

On peut avancer l'hypothèse selon laquelle la survie des micro-organismes est sous la dépendance de la disponibilité de l'eau qui jouerait le rôle de réactif en metant en solution les composés solubles ou partiellement solubles présents dans le milieu.

La mobilité de ces composés, par suite de phénomène d'osmose, entrainerait la destruction des micro-organismes.

Les travaux de la demanderesse ont, en plus, montré que l'évolution des isothermes de sorption des

**0 083 267**

inoculums, et par là l'activité de l'eau, était modifiée par le mode d'obtention de l'isotherme (phénomène d'hystérésis), par la température, par la nature du gel, par la nature et la concentration des sources hydrocarbonées et des substances solubles du milieu de culture, par la modification du milieu de culture par le micro-organisme, par les phénomènes de cristallisation des composés cristallisables présents.

Ces travaux ont montré, en outre, les caractéristiques spécifiques d'hygroscopicité de différents polymères utilisés selon leur nature ou leur mode d'obtention.

Le polymère utilisé est avantageusement du groupe des polysaccharides auquel on fait subir un traitement de réticulation au moins partiel.

Par traitement de réticulation au moins partiel, on entend un traitement susceptible de modifier la structure du polysaccharide, tel que traitement thermique, traitement par un sel métallique ou alcalino-terreux ou au moyen d'un autre polymère et de préférence par un autre polysaccharide.

Le sel métallique est tel que de fer ou d'aluminium.

Le sel alcalino-terreux est tel que de calcium.

Avantageusement le polymère est à base d'un hétéro-polysaccharide à haut poids moléculaire obtenu par fermentation d'un hydrate de carbone par un micro-organisme du genre Xanthomonas ou Arthrobacter ou par des champignons appartenant au genre Sclerotium.

On peut également faire appel à des polysaccharides issus de gommes naturelles ou biosynthétiques, de provenance diverses: algues (alginates, carraghénanes, agar), exsudats de plantes (gommes karaya, adragante, arabique) et de graines (guar, caroube).

Comme déjà dit, avatageusement on fait appel à des polymères à base de polysaccharides qui vont influer sur le devenir du micro-organisme dans le sol suite à l'inoculation:

— la taille des particules de polymère permet la meilleure dissémination des Rhizobium.
— la poudre de polysaccharide se réhumidifie rapidement;
— le polysaccharide se lie aux particules de sols et autres substrats et aux racines;
— le polysaccharide est rapidement dégradé dans le sol, les Rhizobium sont libérés de leur matrice de polymère et infectent les racines de la légumineuse.

Selon l'invention, le milieu de culture renferme au moins une source hydrocarbonée. Celle-ci est telle que du groupe des sucres, des polyols et des polysaccharides. On peut citer par exemple comme source hydrocarbonée le mannitol, le glucose, la dextrine, l'amidon.

La source hydrocarbonée doit comporter de préférence des sucres supérieurs à $C_6$ et tout particulièrement le mannitol, le sorbitol ou le fructose.

Le milieu de culture renferme également avantageusement au moins un sel minéral.

Le milieu de culture renferme également une source azotée minérale organique telle que l'extrait de levure par exemple de composition suivante:

| | |
|---|---|
| Amino | 5,5 |
| NaCl | 0,5 |
| Ca | 0,08 |
| Fe | 0,20 |
| K | 3,4 |
| Mg | 0,07 |
| P | 1,16 |
| Hydrates de carbone | 16,6 |
| Arginine | 3,5 |
| Cystine | 1,6 |
| Histidine | 1,5 |
| Isoleucine | 4,7 |
| Leucine | 6,4 |
| Lysine | 6,5 |
| Methionine | 2,0 |
| Phénylalanine | 3,5 |
| Thréonine | 3,3 |
| Tryptophane | 1,0 |
| Tyrosine | 4,0 |
| Valine | 4,8 |
| Vitamines | 2,7 cg/g |

Il est à noter que la source azotée ne contient pas de molécules de haut poids moléculaire. Sa concentration dans le milieu avant toute culture est de l'ordre de 0,1% mais l'extrait est partiellement consommé au cours de la culture.

L'ensemble gel, sel minéral, source hydrocarbonée et source azotée constitue de préférence entre 2 et 10% en poids de la suspension à sécher.

4

Le polymère représente de préférence entre 1 et 2% de la suspension à sécher.

La source hydrocarbonée représente préférentiellement entre 0,5% et 1% et l'extrait de levure entre 0,05% et 0,1% du milieu de culture.

On peut ajouter à la solution avant séchage des additifs tels que ceux précédemment cités ou tout autre produit inerte tout en restant dans les limites précédemment données (2 à 10% de la suspension à sécher).

Le micro-organisme utilisé dans la présente invention est notamment du genre Rhizobium.

Parmi les micro-organismes du genre Rhizobium utilisés pour la présente invention, on peut citer: Rhizobium Japonicum, Rhizobium meliloti, Rhizobium phaseoli, Rhizobium leguminosarum, Rhizobium trifolii.

Les Rhizobium sont parmi les micro-organismes les plus sensibles à la température et à la deshydratation. Il est connu que les bactéries gram négatif sont particulièrement sensibles à la dessication (Soil Biol. Biochem. Vol. 14 p. 13—14, 1982), et les Rhizobium confirment cette affirmation.

Parmi les espèces de Rhizobium, le Rhizobium Japonicum USDA 138 a été décrit par F. Munévar (Applied and Environmental Microbiology, Aug. 1981 p. 272—276) comme ayant une température maximale de survie de 39,1° Celsius.

Ce micro-organisme contrairement à de nombreux autres qui sont thermorésistants comme par exemple les Bacillus ne peut à la lecture de l'art antérieur être séché à haute température sans risque.

Le milieu de culture privilégié utilisé pour la croissance du Rhizobium Japonicum est le milieu YEM, milieu classique bien connu de l'homme de l'art.

La concentration bactérienne peut être augmentée par centrifugation préalable du milieu de culture et remise en suspension du culot bactérien.

Le milieu de culture ou la suspension bactérienne est ensuite apporté à un gel de polysaccharide dans lequel le micro-organisme est inclus, puis le gel est séché.

On peut aussi, selon une autre forme de mise en oeuvre, dissoudre le polysaccharaide dans le milieu de culture.

Le séchage peut se faire de diverses manières, en une ou plusieurs étapes.

Selon l'invention, un simple contact à l'air dans les conditions habituelles de température et d'humidité relative n'est pas suffisant.

L'on doit faire appel des techniques qui permettent d'obtenir une dessication plus poussée telles que:

— flux d'air chaud et sec, évidemment dans les conditions compatibles avec la survie du micro-organisme.

— solution saturée, en un composé présentant l'activité de l'eau requise, évoluant vers l'équilibre avec l'inoculum.

Par ailleurs, on peut apporter l'inoculum sur un support tel que à base de silice.

Dans le cas où l'on fait appel à une méthode impliquant un absorbant tel que la silice, cet absorbant peut en plus remplir une fonction de support et charge inerte, facilitant la mise en oeuvre et la manipulation de l'inoculum.

On peut en particulier faire appel à une technique de séchage par atomisation, qui, de manière inattendue conduit à des inoculums dans lesquels la survie du micro-organisme est préservée alors même que les températures des gaz de traitement sont largement supérieures à celles que le micro-organisme peut subir.

Dans le cas de l'atomisation, le gel à sécher doit posséder des propriétés de »coulabilité«.

Dans ce cas éventuellement, la réticulation du polymère peut être provoquée par le traitement thermique lors du séchage.

Bien évidemment, on peut combiner plusieurs de ces techniques.

Du point de vue économique, il est préférable d'adopter un système de séchage bi-étagé comme celui utilisé pour la deshydratation du lait. Ce système se compose d'un séchage par atomisation qui lorsqu'il est effectué avec une température de sortie d'environ 95°C conduit à une activité de l'eau de l'ordre de 0,2 à 0,4 puis un séchage par lit fluidisé jusqu'à une activité de l'eau inférieure à 0,1.

L'utilisation d'un système bi-étagé permet d'augmenter le débit de la substance à sécher, par conséquent l'écart de température entre la sortie et l'entrée. La solution entrant dans l'atomiseur peut contrairement à un préjugé établi être soumise à une température de 250°C et plus sans aucune perte de survie des micro-organismes. La température de sortie est maintenue au-dessus du point de rosée ce qui évite une rehydratation ds produits. Cette température est notamment de l'ordre de 75°C à 95°C.

Le lit fluidisé utilisé pour terminer le séchage et atteindre une activité de l'eau inférieure à 0,1 consomme 2 à 4 fois moins d'énergie pour l'élimination de la dernière fraction d'eau que l'atomisation. Globalement l'économie d'énergie réalisée par rapport à un atomiseur simple est de l'ordre de 20%.

Les polymères séchés peuvent se présenter sous diverses formes: gels, billes, fibres.

Avantageusement, on cherche à présenter les inoculums obtenus sous forme de poudre ou microgranulés pour faciliter leur mise en oeuvre.

La limitation des transferts d'eau entre l'inoculum polymère à faible activité de l'eau et l'atmosphère environnante doit être réalisée par le choix d'un mode d'emballage adapté.

La caractéristique principale de l'emballage sélectionné est son imperméabilité à la vapeur d'eau. Cela suppose que l'emballage ait été parfaitement réalisé, c'est-à-dire qu'il ne présente ni microporo sité, ni microcanaux de fuite aux soudures. Dans l'emballage, il s'établit un équilibre de pression d'eau entre l'atmosphère interne et l'inoculum; une fois l'équilibre atteint, la capacité d'absorption d'eau de l'inoculum devient négligeable (rapport kg d'air/kg de produit très faible). Pour le type de protection que l'on veut assurer, les films de polypropylène correspondent à un matériau d'emballage approprie tel que Pryphane commercialisé par R. P. Films. Les films de polypropylène assurent une bonne stabilité thermique (non conductivité, non réflectivité), une faible transmission de la lumière, une bonne perméabilité aux gaz.

Il est possible d'associer un deshydratant dans le sachet d'emballage.

Au moyen de la présente invention, il a été possible de conserver des Rhizobium pendant une période d'au moins deux années sans aucune perte décelable.

Un avantage de l'invention est de pouvoir insérer dans l'inoculum des produits du type antifongi ques sans aucune altération de la cellule bactérienne. Les produits pouvant être utilisés à l'intérieur de l'inoculum sont notamment du genre thirame, éthyl phosphite d'aluminium. A des activités de l'eau inférieures à 0,1 il n'y a aucune perte due à la présence de l'antifongique.

Tableau I

| Traitements | Log du nombre de Rhizobium/g après 30 jours de stockage (25° C) Activité de l'eau | | | | | |
|---|---|---|---|---|---|---|
| | 0,06 | 0,22 | 0,39 | 0,43 | 0,52 | 0,75 |
| Inoculum A sans thirame | 7,82 | 7,74 | 7,43 | 7,39 | 0 | 0 |
| Inoculum A avec thirame dans les proportions (1/2) | 8,83 | 8,62 | 8,04 | 7,97 | 5,65 | 0 |
| Inoculum A éthyl phosphite d'aluminium (1/1) | 8,81 | 0 | 0 | 0 | 0 | 0 |

thirame Disulfure de bis (biméthyl-thiocarbamyl) m. a. 90% — solubilité: 30 ppm.
Log du nombre de Rhizobium/g d'inoculum avant stockage: 9,04.

Preparations des inoculums

I°) Préparation d'un inoculum gel Xanthane-caroube

On obtient les inoculums suivants:

— un inoculum A, Xanthane-caroube-mannitol si le mannitol a été utilisé comme source hydrocarbo née du milieu de culture;
— ou un inoculum B, Xanthane-caroube-glycérol si le glycérol a été utilisé comme source carbonee du milieu de culture;
— ou un inoculum C Xanthane-caroube-DE-40 (dextrine à 40% de fonction réductrice rendue par les laboratoires Roquette) si le sirop de glucose DE-40 a été utilisé comme source carbonée du milieu de culture;
— ou un inoculum D, Xanthane-caroube DE-33 (dextrine à 33% de fonction réductrice rendue par les laboratoires Roquette) si le sirop de glucose DE-33 a été utilisé comme source hydrocarbonée du milieu de culture;
— ou un inoculum E, Xanthane-caroube dextrine, si la dextrine a été utilisée comme source hydrocar bonée du milieu de culture;
— ou un inoculum F, Xanthane-caroube-amidon, si de l'amidon soluble a été utilisé comme source carbonée du milieu de culture.

Mode de préparation de l'inoculum A

— milieu de culture — milieu YEM
— mannitol     10,0 g/l
— extrait de levure DIFCO     1,0 g/l
— $K_2HPO_4$     0,5 g/l
— $MgSO_4, 7 H_2O$     0,2 g/l
— $FeCl_3$     0,004 g/l
— NaCl     0,2 g/l
— le pH est ajusté à 6,8 avec HClN
— stérilisation 120°C

— selon une variante on remplace le mannitol par une autre source carbonée (inoculum B, C, D, E ou F).
— la souche utilisée de Rhizobium japonicum est la souche $G_3$ USDA 138 Beltswille dans laquelle le nombre de Rhizobium par ml de culture après incubation (6 jours) est de l'ordre de $2-3 \cdot 10^9$ avec le mannitol ou les différentes sources carbonées utilisées.

On prépare deux solutions (les valeurs sont données pour la préparation de 300 g d'inoculum).
a) Solution de polysaccharide de type anionique résultant de la fermentation d'hydrates de carbone par un micro-organisme de genre Xanthomonas de $PM > 2 \cdot 10^6$, que l'on désignera par produit 1.
On amène 100 ml d'eau distillée à 70—80°C, on ajoute 1,5 g du produit 1, on maintient cette température 20 à 30 minutes, sous agitation puis on le ramène entre 40 et 50°C.
b) Solution de farine de graine de caroube = polysaccharide constitué d'unités $\beta$-D-manno-pyrano-syl (liaisons 1→4) une sur quatre ou cinq étant substituée en $C_6$ par un $\alpha$-D-galacto-pyranosyl $PM = 3,1 \cdot 10^5$ que l'on désignera par la suite par produit 2.
On procède de la même façon en remplaçant le produit 1 par 1,5 g de farine de graine de caroube (produit 2).
Lorsque les deux solutions sont à 40—45°C, on ajoute, sous agitation, à chacune d'elle, 50 ml de la culture bactérienne. On verse alors, en agitant vigoureusement le mélange culture + polysaccharide dans le mélange culture + farine de graine de caroube. L'obtention d'un gel consistant est instantanée si les deux solutions sont mélangées vigoureusement.

## 2°) Préparation d'un inoculum polymère alginate $CaCl_2$

On obtient un inoculum G (alginate-$CaCl_2$) si on utilise le mannitol comme source du milieu YEM.
Trois étapes sont nécessaires.
a) Préparation de la suspension de Rhizobium dans l'alginate.
On dissout dans 100 ml de culture bactérienne 2 g d'alginate de sodium faible viscosité $(0,130-0,240$ Pa $\cdot$ s).
b) Gélification. On fait tomber la suspension de Rhizobium dans l'alginate dans une solution de $CaCl_2 \cdot 2 H_2O$ (170 g/l). La gélification se fait en billes si l'on fait tomber goutte à goutte la suspension dans la solution de $CaCl_2 \cdot H_2O$ en agitant cette solution. La gélification se fait en fibres si l'on verse 20 ml de la solution de $CaCl_2$, sous agitation, dans la solution d'alginate.
c) Lavage. Aussitôt après la gélification, on lave l'inoculum polymère en billes (ou en fibres), dans l'eau courante.

## 3°) Préparation d'un inoculum polymère alginate $CaSO_4$ (gel)

On obtient un inoculum H alginate $CaSO_4$ si on utilise le mannitol comme source carbonée du milieu YEM.
Deux étapes sont nécessaires:
a) Préparation de la suspension de Rhizobium dans un alginate de viscosité comprise entre 0,75 et 1 Pa $\cdot$ s. On dissout dans 80 ml de culture bactérienne 1 g d'alginate. Pour cela, on disperse la poudre en pluie fine sur la culture en l'agitant continuellement jusqu'à solubilisation complète de l'alginate.
b) Gélification. On ajoute à la suspension de Rhizobium dans l'alginate 20 ml d'une solution de $CaSO_4 \cdot 2 H_2O$ à 6 g/l. La prise en masse du gel est instantanée.
Dans les exemples suivants, on a réglé l'activité de l'eau au moyen de solutions saturées dont les valeurs des activités de l'eau $a_w$ à une température de 25°C sont exposées dans le tableau II.

7

Tableau II

| Solutes (Solutions saturées) | Valeur de $a_w$, 25°C | |
| --- | --- | --- |
| NaOH | 0,0695 | |
| Actigel | 0,11 | |
| $KC_2H_3O_2$ (1,5 $H_2O$) | 0,226 | |
| $MgCl_2$ | 0,3273 | 0,332 |
| $CrO_3$ | 0,396 | |
| $K_2CO_3$ $2H_2O$ | 0,438 | 0,4276 |
| $Mg(NO_3)_2$ | 0,5288 | |
| $Na_2Cr_2O_7$ | 0,535 | |
| NaBr | 0,5770 | |
| $CuCl_2$ | 0,886 | |
| $NaNO_3$ | 0,7373 | |
| NaCl | 0,7532 | |
| KCl | 0,8432 | |
| $KNO_3$ | 0,920 | |
| $NH_4H_2PO_4$ | 0,927 | |

On a illustré dans les divers tableaux et courbes l'influence d'un certain nombre de paramètres.

## Exemple 1

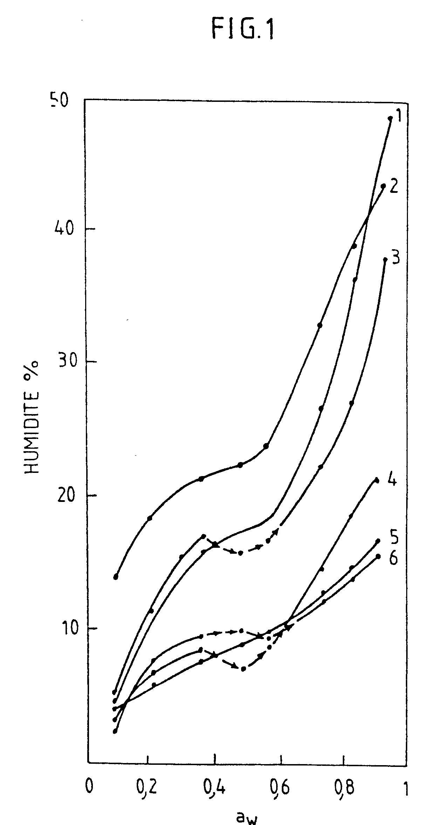

Il représente la valeur de l'activité de l'eau en relation avec le taux d'humidité pour différents types d'inoculums en fonction du gel et de la source hydrocarbonée (figure 1).

| N° Courbe et type d'inoculum | Source hydrocarbonée | Gel | Type de séchage |
| --- | --- | --- | --- |
| 1 (B) | Glycérol | Gomme Xanthane Caroube | à l'air |
| 2 (G) | Mannitol | Alginate | à l'air |
| 3 (A) | Mannitol | Xanthane Caroube | à l'air |
| 4 | Mannitol | sans | Lyophilisation |
| 5 | Mannitol | sans | Deshydratation sous vide |
| 6 | Mannitol | sans | sour tourbe |

Les petites flèches montrent le phénomène de cristallisation du mannitol.

## Exemple 2

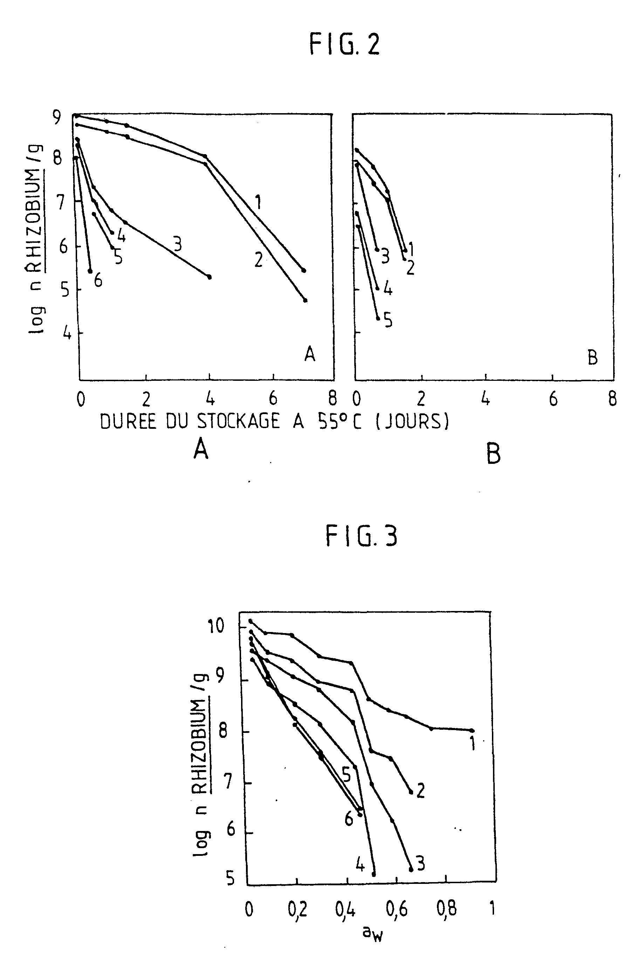

Il montre l'effet de la température (55°C) sur la survie des Rhizobium au cours du stockage dans l'inoculum A (figure 2A) contenant comme source hydrocarbonée le mannitol et dans l'inoculum B (figure 2B) contenant comme surce hydrocarbonée le glycérol en fonction de l'activité de l'eau (tracé 1, $a_w = 0,09$; tracé 2, $a_w = 0,11$; tracé 3, $a_w = 0,22$; tracé 4, $a_w = 0,32$; tracé 5, $a_w = 0,43$; tracé 6, $a_w = 0,52$).

Ces deux figures montrent que la conservation des micro-organismes est bien meilleure avec un sucre en $C_6$ qu'avec un sucre en $C_3$.

## Exemple 3

Il représente les courbes de survie du Rhizobium dans l'inoculum A (xanthane caroube et mannitol) en fonction de l'$a_w$ après différents temps de stockage à 25°C (tracé 1 = 10 j; tracé 2 = 20 j; tracé 3 = 30 j; tracé 4 = 80 j; tracé 5 = 110 j; tracé 6 = 180 j) (Figure 3).

## Exemple 4

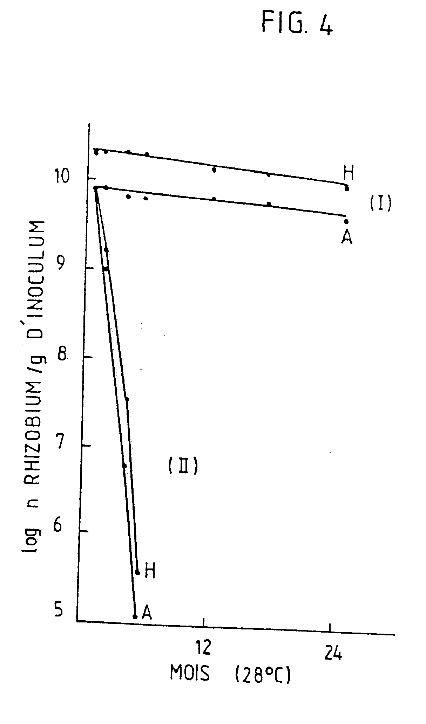

Il représente la survie du Rhizobium au cours du stockage à 28°C dans l'inoculum A (xanthane caroube et mannitol) et dans l'inoculum H (alginate et mannitol) en fonction du temps et de différents $a_w$, courbe I $a_w = 0,06$, courbe II $a_w = 0,42$ (figure 4).

Elle permet de voir qu'il n'existe aucune différence significative dans la survie du Rhizobium entre les différents polysaccharides.

## Exemple 5

Il illustre les courbes de survie du Rhizobium en fonction de la source hydrocarbonée présente dans le milieu de culture.

```
tracé  A  =  mannitol
tracé  B  =  glycérol
tracé  C  =  glucose DE 40
tracé  D  =  glucose DE 33
tracé  E  =  dextrine
tracé  F  =  amidon
```

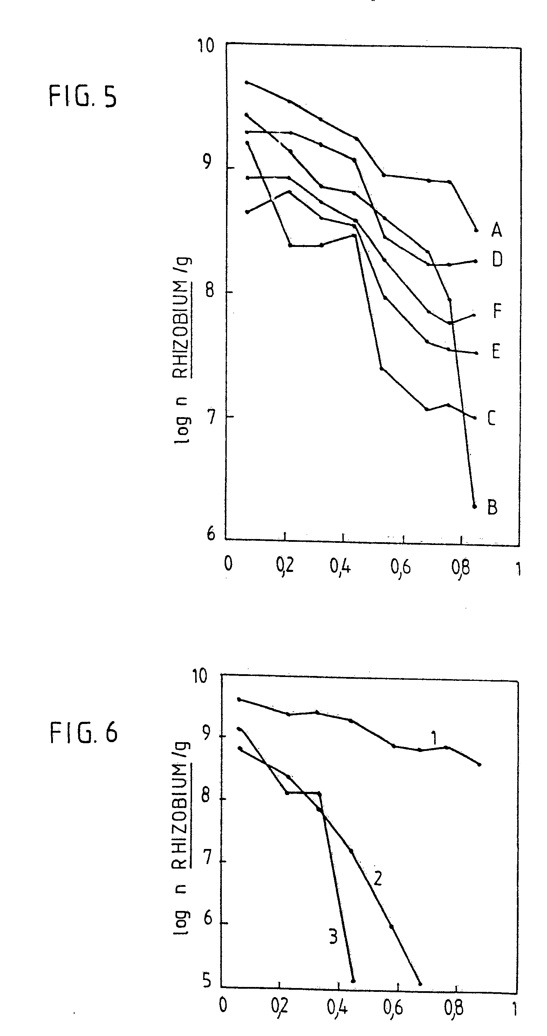

pendant un stockage de 10 jours à 28°C aux différents $a_w$ (Figure 5).

## Exemple 6

Il représente les courbes de survie de trois souches de Rhizobium inclus dans le polymère A après 10 jours de stockage (28°C) (tracé 1 Rhizobium Japonicum USDA 138 Beltswille; tracé 2 Rhizobium meliloti 2011, INRA DIJON; tracé 3, Rhizobium phaseoli souche Olivia Université Minnesota, USA) (Figure 6).

## Exemple 7

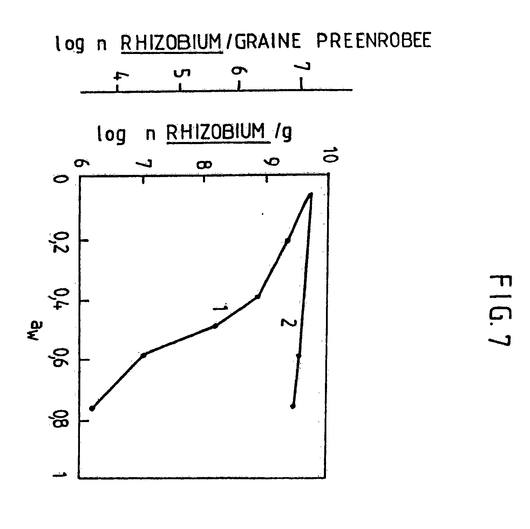

Il représente les courbes de survie du Rhizobium japonicum dans l'inoculum A pour les graines de soja (ex. Kingsoy) préenrobées en fonction de l'$a_w$ et à deux températures (tracé 1, 28°C; tracé 2, 4°C) après 60 jours de stockage (Figure 7).

## Exemple 8

On a séché les inoculums A et H dans un atomiseur tel que décrit dans l'ouvrage de Masters Spray Drying — second edition John Wiley & Sons 1976 —, en mettant en oeuvre un atomiseur à turbine.

En utilisant une température de sortie de 75°C et en faisant varier la température d'entrée entre 150 et 250°C, on a pu constater que le log n Rhizobium vivant par gramme de poudre atomisée demeurait stable et égal à la valeur de départ c'est-à-dire $10^{10}$ Rhizobium par gramme. Il n'y a donc pas destruction du Rhizobium et l'on peut obtenir une présentation en poudre.

## Revendications

1. Procédé de préparation d'inoculum à longue viabilité et à résistance à la température améliorée, caractérisé en ce que on inclut le(s) micro-organisme(s) dans son milieu de culture dans un gel de polysaccharide et que l'on abaisse l'activité de l'eau dans l'inoculum en-dessous de 0,1 et qu'on la maintient à cette valeur.

2. Procédé selon la revendication 1, caractérisé en ce que le polysaccharide est réticulé par l'un des modes de traitement suivants: thermique, à l'aide d'un sel métallique au moyen d'un autre polymère.

3. Procédé selon la revendication 1, caractérisé en ce que le polymère est un hétéropolymère à base de gomme xanthane et de farine de caroube.

4. Procédé selon la revendication 1, caractérisé en ce que le polymère est un polymère à base d'alginate.

5. Procédé selon la revendication 1, caractérisé en ce que le milieu de culture contient une source hydrocarbonée choisie parmi les sucres, les polyols et les polysaccharides.

6. Procédé selon la revendication 5, caractérisé en ce que la source hydrocarbonée est choisie parmi les sucres, les polyols d'au moins 6 carbones.

7. Procédé selon la revendication 6, caractérisé en ce que la source hydrocarbonée est du groupe mannitol, sorbitol, fructose, dextrine et amidon.

8. Procédé selon l'une des revendications 1 à 7, caractérisé en ce que le milieu de culture renferme une source azotée minérale ou organique telle que l'extrait de levure.

9. Procédé selon la revendication 1, caractérisé en ce que le milieu de culture renferme au moins un sel minéral.

10. Procédé selon les revendications 1 à 9, caractérisé en ce que le micro-organisme est du genre Rhizobium.

11. Procédé selon la revendication 10 caractérisé en ce que le micro-organisme est le Rhizobium Japonicum.

12. Procédé selon la revendication 1, caractérisé en ce que l'activité de l'eau est abaissée par atomisation.

13. Procédé selon la revendication 1, caractérisé en ce que l'activité de l'eau est abaissée par atomisation et passage sur un lit fluidisé.

14. Procédé selon la revendication 1, caractérisé en ce que l'on insère dans l'inoculum un antifongique.

15. Inoculum obtenable selon le procédé des revendications 1 à 11, caractérisé en ce que le polymère est du groupe des polysaccharides au moins partiellement réticulé, que la source hydrocarbonée est du groupe du mannitol, sorbitol, fructose, glucose, dextrine, amidon et que le micro-organisme est un Rhizobium.

16. Application de l'inoculum selon la revendication 15 au préenrobage des graines.

17. Application de l'inoculum selon la revendication 15 à l'inoculation du sol.

## Patentansprüche

1. Verfahren zur Herstellung eines Inoculum von langer Lebensfähigkeit und verbesserter Temperaturbeständigkeit, dadurch gekennzeichnet, daß man den Mikroorganismus/die Mikroorganismen in seinem/ihrem Kulturmedium in ein Polysaccharidgel einschließt und daß man die Aktivität des Wassers im Inoculum auf unter 0,1 verringert und bei diesem Wert hält.

2. Verfahren nach Anspruch 1, dadurch gekennzeichnet, daß das Polysaccharid durch eine thermische Behandlung, Behandlung mit einem Metallsalz oder mit Hilfe eines anderen Polymeren vernetzt worden ist.

3. Verfahren nach Anspruch 1, dadurch gekennzeichnet, daß das Polymer ein Heteropolymer auf der Basis von Xanthangummi und Johannisbrotkernmehl ist.

4. Verfahren nach Anspruch 1, dadurch gekennzeichnet, daß das Polymer ein Polymer auf Alginatbasis ist.

5. Verfahren nach Anspruch 1, dadurch gekennzeichnet, daß das Kulturmedium eine Kohlenwasserstoffquelle, ausgewählt unter den Zuckern, Polyolen und Polysacchariden, enthält.

6. Verfahren nach Anspruch 5, dadurch gekennzeichnet, daß die Kohlenwasserstoffquelle ausgewählt wird unter den Zuckern und Polyolen mit mindestens 6 Kohlenstoffatomen.

7. Verfahren nach Anspruch 6, dadurch gekennzeichnet, daß die Kohlenwasserstoffquelle zur Gruppe Mannit, Sorbit, Fructose, Dextrin und Stärke gehört.

8. Verfahren nach einem der Ansprüche 1 bis 7, dadurch gekennzeichnet, daß das Kulturmedium eine anorganische oder organische Stickstoffquelle wie Hefeextrakt enthält.

9. Verfahren nach Anspruch 1, dadurch gekennzeichnet, daß das Kulturmedium mindestens ein anorganisches Salz enthält.

10. Verfahren nach den Ansprüchen 1 bis 9, dadurch gekennzeichnet, daß der Mikroorganismus zur Gattung Rhizobium gehört.

11. Verfahren nach Anspruch 10, dadurch gekennzeichnet, daß der Mikroorganismus Rhizobium Japonicum ist.

12. Verfahren nach Anspruch 1, dadurch gekennzeichnet, daß die Aktivität des Wassers durch Zerstäuben verringert wird.

13. Verfahren nach Anspruch 1, dadurch gekennzeichnet, daß die Aktivität des Wassers durch Zerstäuben und Überleiten über ein Wirbelbett verringert wird.

14. Verfahren nach Anspruch 1, dadurch gekennzeichnet, daß man in das Inoculum ein Antipilzmittel einschließt.

15. Inoculum, erhältlich durch das Verfahren der Ansprüche 1 bis 11, dadurch gekennzeichnet, daß das Polymer zur Gruppe der zumindest teilweise vernetzten Polysaccharide gehört, die Kohlenwasserstoffquelle zur Gruppe Mannit, Sorbit, Fructose, Glucose, Dextrin und Stärke gehört und der Mikroorganismus ein Rhizobium ist.

16. Anwendung des Inoculum nach Anspruch 15 zum Vorumhüllen von Samenkörnern.

17. Anwendung des Inoculum nach Anspruch 15 zum Beimpfen des Bodens.

## Claims

1. A process for the preparation of inoculum having long-term viability and enhanced temperature resistance characterised by including the micro-organism(s) in its culture medium in a polysaccharide gel, lowering the activity of water in the inoculum to below 0.1, and maintaining it at that value.

2. A process according to claim 1 characterised in that the polysaccharide is cross-linked by one of the following forms of treatment: thermal, with a metal salt, by means of another polymer.

3. A process according to claim 1 characterised in that the polymer is a heteropolymer based on xanthan gum and carob flour.

4. A process according to claim 1 characterised in that the polymer is an alginate base polymer.

5. A process according to claim 1 characterised in that the culture medium contains a hydrocarbon source selected from sugars, polyols and polysaccharides.

6. A process according to claim 5 characterised in that the hydrocarbon source is selected from sugars and polyols having at least six carbons atoms.

7. A process according to claim 6 characterised in that the hydrocarbon source is from the group comprising mannitol, sorbitol, fructose, dextrin and starch.

8. A process according to one of claims 1 to 7 characterised in that the culture medium contains an organic or inorganic nitrogenbearing source such as yeast extract.

9. A process according to claim 1 characterised in that the culture medium contains at least one mineral salt.

10. A process according to claims 1 to 9 characterised in that the micro-organism is of the genus Rhizobium.

11. A process according to claim 10 characterised in that the micro-organism is Rhizobium Japonicum.

12. A process according to claim 1 characterised in that the activity of the water is reduced by atomisation.

13. A process according to claim 1 characterised in that the activity of the water is reduced by atomisation and by passing it over a fluidised bed.

14. A process according to claim 1 characterised in that a fungicide is inserted in the inoculum.

15. An inoculum which can be produced in accordance with the process of claims 1 to 11 characterised in that the polymer is of the group of at least partially cross-linked polysaccharides, that the hydrocarbon source is from the group comprising mannitol, sorbitol, fructose, glucose, dextrin and starch, and that the micro-organism is Rhizobium.

16. Use of the inoculum to claim 15 for pre-encasing seeds.

17. Use of the inoculum according to claim 15 for the inoculation of the ground.

# FIG.1

# FIG. 2

DUREE DU STOCKAGE A 55°C (JOURS)

# FIG. 3

FIG. 4

FIG. 5

FIG. 6

FIG. 7